# EUROPÄISCHE PATENTSCHRIFT

(11) **EP 1 232 733 B1**
(45) Veröffentlichungstag und Bekanntmachung des Hinweises auf die Patenterteilung: **02.01.2004**
(21) Anmeldenummer: 02001620.0
(22) Anmeldetag: 23.01.2002
(51) Int. Cl.: A61F 2/52

(54) **Brustprothese**
Breast prosthesis
Prothese mammaire

(30) Priorität: 23.01.2001 DE 20101174 U
(43) Veröffentlichungstag der Anmeldung: 21.08.2002
(73) Patentinhaber: Amoena Medizin-Orthopädie-Technik GmbH & Co., 83064 Raubling (DE)
(72) Erfinder: Stelter, Nils, 83101 Achenmühle (DE)
(74) Vertreter: Laufhütte, Dieter, Dr.-Ing.

(56) Entgegenhaltungen:
- EP-A- 0 768 068
- DE-C- 4 421 516
- US-A- 3 075 529
- US-A- 4 856 294

## Beschreibung

Die Erfindung betrifft eine Brustprothese nach dem Oberbegriff des Anspruchs 1.

Beispielsweise aus der DE 27 01 627 ist ein Verfahren zur Herstellung von Brustprothesen aus in Kunststoffolien eingeschweißten, der Brustform nachgebildeten schalenförmigen Körpern aus einer additionsvernetzenden Zweikomponenten-Siliconkautschuk-Masse bekannt. Die mit diesem Verfahren hergestellten Prothesen sind in ihrem Aussehen und in ihrem Verhalten wegen der elastischen Weichheit, der Beweglichkeit, der Konsistenz sowie des Gewichts des verwendeten Materials der natürlichen Brust in nahezu idealerweise nachgebildet. Die Brustprothesen werden möglichst unverrutschbar an der Brust der Trägerin befestigt. Hierzu ist es beispielsweise aus der EP 392 960 bekannt, Brustprothesen der eingangs angegebenen Art innerhalb eines umlaufenden lippenförmigen Randes auf ihrer Rückseite mit einer umlaufenden, durch einen Absatz gebildeten Schulter zu versehen, auf der Haftstreifen oder Haftstücke befestigt sind, die mit den Haftbereichen von an den Körper der Frau durch hautfreundliche Klebemittel befestigte Streifen in der Weise zusammenwirken, daß die Prothese mit den auf der Haut klebenden Haltestreifen verbunden ist und von diesen wieder gelöst werden kann. Als Befestigungsmittel ist dabei beispielsweise eine Klettverbindung vorgesehen.

Gerade wenn die Brustprothese bestimmungsgemäß gut und möglichst unverrutschbar an der Brust der Trägerin befestigt ist, ergibt sich oftmals das Problem eines unangenehmen Wärmestaus unter der Prothese. Die Gründe dafür sind die mangelnde Belüftung, der schlechte Wärmetransport der verwendeten Kunststoffmaterialien der Prothese. Um den Tragekomfort zu verbessern, ist es beispielsweise bereits aus der DE 44 21 516 C1 bekannt geworden, eine Brustprothese mit textiler Rückseite zu schaffen, bei der ein Hohlraum zwischen der textilen Rückseite und der aus Kunststoff bestehenden Prothese mit Watte aufgefüllt werden kann. Diese Lösung führt aber auch nicht zu dem gewünschten Tragekomfort.

Aufgabe der vorliegenden Erfindung ist es daher, eine Brustprothese der eingangs genannten Gattung derart weiterzubilden, daß der Tragekomfort durch Vermeidung des Wärmestaus zwischen der Prothese und der Haut der Trägerin weithin unterdrückt wird.

Diese Aufgabe wird erfindungsgemäß ausgehend von einer Brustprothese nach dem Oberbegriff des Anspruchs 1 durch die Kombination mit den kennzeichnenden Merkmalen des Anspruchs 1 gelöst.

Demnach besteht die Brustprothese zumindest auf ihrer dem Körper zugewandten Seite wenigstens teilweise aus einem sogenannten PCM-Material (Phase Change Material), das eine Phasenumwandlungstemperatur im Bereich der Körpertemperatur aufweist. Das PCM-Material ist während des Phasenwechsels, z.B. vom festen in den flüssigen Zustand oder von dem flüssigen Zustand in die Dampfphase in der Lage, eine bestimmte Wärmemenge bei konstanter Temperatur zu absorbieren. Diese Wärmemenge wird auch als Latentwärme bezeichnet. Beim umgekehrten Phasenwechsel von Dampf in flüssig oder von flüssig in fest, wird die gespeicherte Latentwärme wieder bei konstanter Temperatur abgegeben. Dabei zeichnen sich diese Wärmespeichermaterialien dadurch aus, daß sie für den Einspeichervorgang in einen Latentwärmespeicher im Gegensatz beispielsweise zum Warmwasserspeicher keine großen Temperaturdifferenzen benötigen, um große Wärmemengen zu speichern.

Daher eignet sich dieses Material ideal dazu, die bei einem Wärmestau unterhalb der Prothese auftretende Wärme bei gleicher Temperatur zu absorbieren bzw. diese gespeicherte Wärme bei Absinken der Umgebungstemperatur wieder bei gleicher Temperatur zurückzugeben.

Bevorzugte Ausführungsvarianten der erfindungsgemäßen Lösung ergeben sich aus den an den Hauptanspruch anschließenden Unteransprüchen.

Demnach kann die gesamte Brustprothese aus einer weitgehend homogenen Schicht bestehen, in der das PCM-Material der Siliconkautschuk-Masse beigemischt ist.

Alternativ kann die Brustprothese als Mehrschichtprothese, vorzugsweise Zweischichtprothese, ausgebildet sein, wobei nur in der körpernahen Schicht das PCM-Material beigemischt ist. Während die körperferne Schicht aus einem üblichen additionsvemetzenden Zweikomponenten-Siliconkautschuk bestehen kann, kann die körpernahe Schicht aus Siliconöl oder anderen thixotrop eingestellten Flüssigkeiten, vernetztem Siliconkautschuk oder Siliconschaum bestehen, in die das PCM-Material jeweils eingebettet ist.

Eine alternative Ausführungsvariante ergibt sich dadurch, daß das PCM-Material als Platte ausgebildet ist, die auf der dem Körper zugewandten Seite auf der Prothese befestigt ist. Dabei kann zwischen dem plattenförmigen PCM-Material und der Prothese ein Luftpolster gebildet sein, das einerseits für einen besseren Luftaustausch und zum anderen zu einem verbesserten Tragekomfort führen kann.

Das PCM-Material kann vorteilhaft aus einer Paraffinsubstanz mit einem Schmelzbereich von ca. 33 bis 37°C bestehen. Die als PCM-Material eingesetzten sogenannten Wärmeparaffine sind speziell für wärmetechnische Anwendungen modifizierte Paraffine. Diese Wärmeparaffine weisen eine spezifische Schmelzwärme bzw. Schmelzenthalpie auf, die mit 180 bis 250 kJ/kg für organische Stoffe sehr hoch ist.

Weitere Einzelheiten und Vorteile der Erfindung ergeben sich aus den in der Zeichnung dargestellten Ausführungsbeispielen.

Es zeigen:
- Fig. 1:: einen Längsschnitt durch eine Brustprothese gemäß einer ersten Ausführungsform der vorliegenden Erfindung,
- Fig. 2:: einen Längsschnitt durch eine Brustprothese gemäß einer zweiten Ausführungsform der vorliegenden Erfindung und
- Fig. 3:: einen Längsschnitt durch eine Brustprothese gemäß einer dritten Ausführungsform der vorliegenden Erfindung.

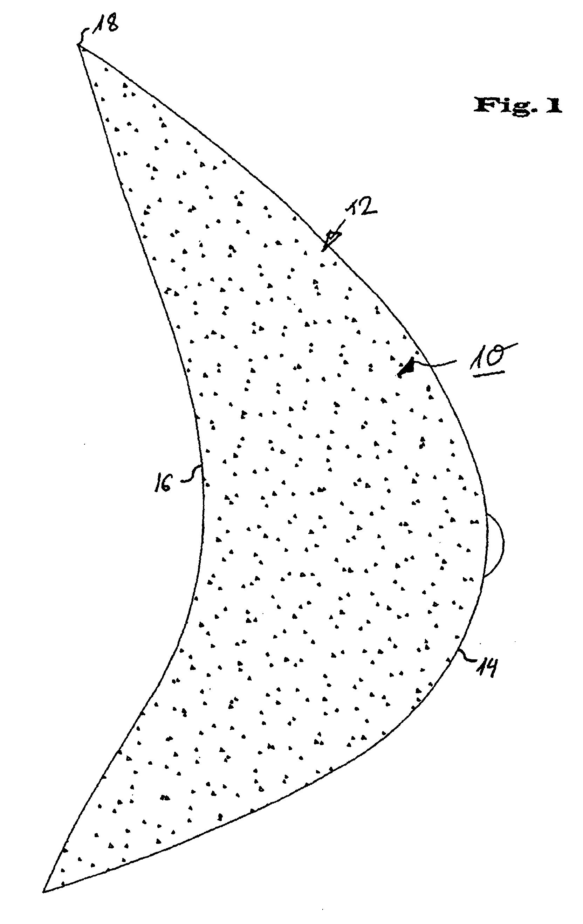

Die in Fig. 1 dargestellte Brustprothese 10 besteht aus einem schalenförmigen Körper 12 aus einer weich-elastisch eingestellten additionsvernetzenden Zweikomponenten-Silikonkautschuk-Masse, dessen Außenseite durch eine Polyurethanfolie 14 und dessen Innenseite durch eine Polyurethanfolie 16 abgedeckt ist, die längs eines gemeinsamen Umfangrandes 18 durch eine umlaufende Schweißnaht miteinander verbunden sind. Im hier dargestellten Ausführungsbeispiel sind in der Zweikomponenten-Siliconkautschuk-Masse gleichverteilt sogenannte Wärmeparaffine, d.h. PCM (Phase Change Material)-Materialien enthalten. Der Schmelzbereich des hier gewählten Wärmeparaffins beträgt ca. 33 bis 37°C. Steigt nun die Hautoberflächentemperatur auf dieses Niveau an, wird das PCM-Material aufgeschmolzen. Hierfür ist entsprechend viel Schmelzenergie notwendig, die als Wärme von der Hautoberfläche abgeführt wird und hier einen angenehmen Kühleffekt bewirkt. Sinkt die Hautoberflächentemperatur zu einem späteren Zeitpunkt wieder unter die Schmelztemperatur des Wärmeparaffins, gibt das Material beim Erstarren die Energie wieder ab. Die Temperaturschwankungen werden somit in einem kleinen Bereich begrenzt und bewirken einen wesentlich verbesserten Tragekomfort.

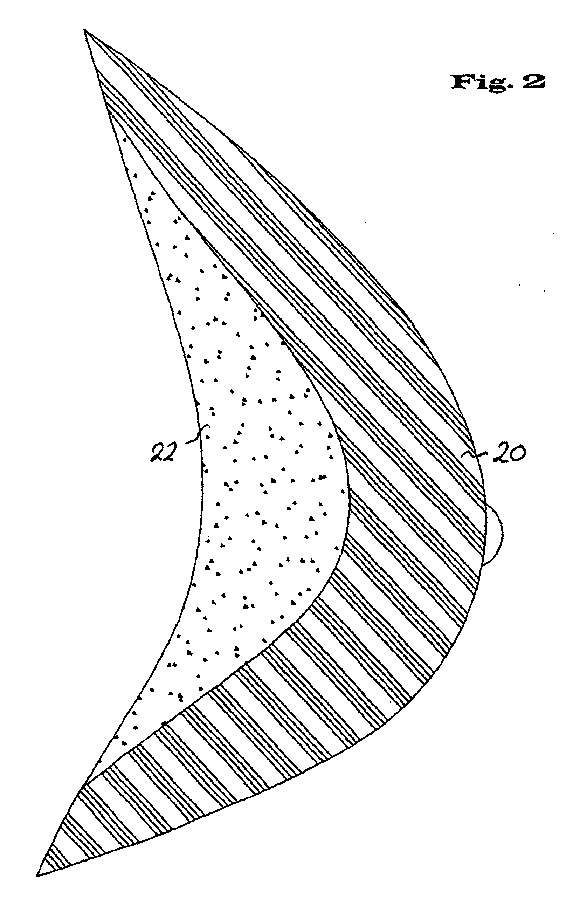

In Fig. 2 ist eine Brustprothese als Zweischicht-Prothese ausgeführt. Die äußere Schicht 20 besteht aus konventionellem additionsvemetzendem Zweikomponenten-Siliconkautschuk, also einem Standardsilicon. Sie kann alternativ auch aus einem sogenannten Lightsilicon bestehen, also einem additionsvernetzendem Zweikomponenten-Siliconkautschuk, dem eine das spezifische Gewicht der Masse erniedrigende Komponente beigemengt ist. Die innere Schicht 22, die dem Körper der Trägerin zugewandt ist, besteht aus einem PCM-Material (Phase Change Material). Das Phase Change Material kann in eingekapselter Form aber auch in reiner Form als Wachs vorliegen. Es kann in Siliconöl oder anderen vorzugsweise thixotropen Flüssigkeiten, vernetztem Silicon oder in einen Schaum eingebettet sein.

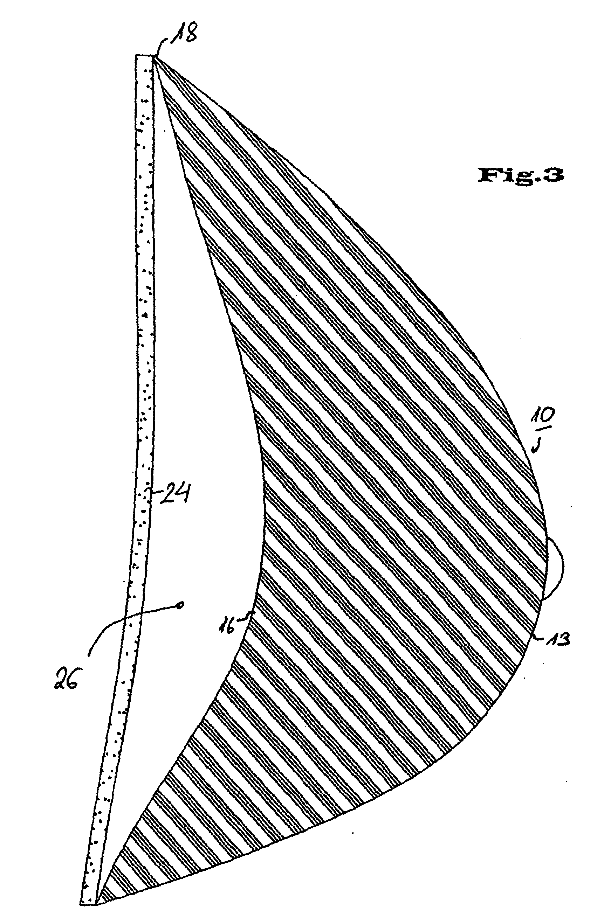

Eine alternative Ausführungsvariante zeigt die Figur 3. Dort ist eine konventionelle Brustprothese 10 in einem Einkammer-Aufbau, wie er grundsätzlich derjenigen gemäß Fig. 1 entspricht, dargestellt. Die Brustprothese 10 ist jedoch hier mit einem konventionellen additionsvernetzendem Zweikomponente-Siliconkautschuk gefüllt. Auf der der Trägerin zugewandten Seite der Brustprothese 10 ist eine als Platte ausgebildete Kühlschicht 24 angeordnet, die vorzugsweise im Bereich des Schweißsaumes 18 der Kunststoffolien 13 und 16 aufgeklebt oder aufgeschweißt ist. Zwischen der plattenförmigen Kühlschicht 24 und der Kunststoffolie 16 ist ein Luftpolster 26 ausgebildet. Die plattenförmige Kühlschicht 24 bzw. Platte besteht aus einem Textil mit integriertem PCM-Material, wie es beispielsweise von der Fa. Schoeller unter der Bezeichnung "Comfortemp" vertrieben wird. Das Luftpolster 26 sorgt für einen verbesserten Tragekomfort.

Die Platte 24 kann alternativ auch aus einem Pad mit PCM-Material bestehen, das auch als Austauschvariante denkbar ist. Hierzu ist die flexibel gestaltete Platte 24 in an sich bekannter Weise lösbar mit der restlichen Prothese im Bereich des Umfangrandes 18 verbunden.

## Patentansprüche

1. Brustprothese (10) im wesentlichen bestehend aus in Kunststoffolien eingeschweißten, der Brustform nachgebildeten Körpern (12) aus einer additionsvernetzenden Zweikomponenten-Siliconkautschuk-Masse,
**dadurch gekennzeichnet,**
**daß** sie zumindest auf ihrer dem Körper zugewandten Seite wenigstens teilweise aus einem PCM-Material besteht, das eine Phasenumwandlungstemperatur im Bereich der Körpertemperatur aufweist.

2. Brustprothese (10) nach Anspruch 1, **dadurch gekennzeichnet, daß** sie aus einer Schicht (20) besteht und daß das PCM-Material der Siliconkautschuk-Masse beigemischt ist.

3. Brustprothese (10) nach Anspruch 1, **dadurch gekennzeichnet, daß** sie als Mehrschichtprothese, vorzugsweise Zweischichtprothese, ausgebildet ist, und daß nur in der körpernahen Schicht (22) das PCM-Material beigemischt ist.

4. Brustprothese (10) nach Anspruch 3, **dadurch gekennzeichnet, daß** in der zweiten körpernahen Schicht das PCM-Material in Siliconöl, anderen vorzugsweise thixotropen Flüssigkeiten, vernetztem Siliconkautschuk oder Siliconschaum eingebettet ist.

5. Brustprothese (10) nach Anspruch 1, **dadurch gekennzeichnet, daß** das PCM-Material als Platte (24) ausgebildet ist, die auf der dem Körper zugewandten Seite auf der Prothese (10) befestigt ist.

6. Brustprothese (10) nach Anspruch 5, **dadurch gekennzeichnet, daß** zwischen dem plattenförmigen PCM-Material und der Prothese ein Luftpolster (26) gebildet ist.

7. Brustprothese (10) nach einem der vorangehenden Ansprüche, **dadurch gekennzeichnet, daß** das PCM-Material eine Paraffinsubstanz ist.

8. Brustprothese (10) nach Anspruch 7, **dadurch gekennzeichnet, daß** das PCM-Material bildende Paraffin in eingekapselter oder stabilisierter Form eingesetzt ist.

9. Brustprothese (10) nach Anspruch 7, **dadurch gekennzeichnet, daß** das als PCM-Material eingesetzte Paraffin in reiner Form (als Wachs) eingesetzt ist.

10. Brustprothese (10) nach einem der Ansprüche 5 bis 9, **dadurch gekennzeichnet, daß** das plattenförmige Material aus einem Textil mit integriertem PCM-Material besteht.

11. Brustprothese (10) nach einem der Ansprüche 5 bis 9, **dadurch gekennzeichnet, daß** die Platte (24) durch eine mit PCM-Material gefüllte Tüte gebildet wird.

## Claims

1. Breast prosthesis (10) consisting principally of bodies (12) which are made of an addition-crosslinked two-component silicone rubber compound, are welded into plastic sheets and simulate the breast shape, **characterized in that**, at least on its side facing towards the body, it is made at least partially of a phase-change material having a phase transition temperature in the region of body temperature.

2. Breast prosthesis (10) according to Claim 1, **characterized in that** it consists of one layer (20), and **in that** the phase-change material is admixed to the silicone rubber compound.

3. Breast prosthesis (10) according to Claim 1, **characterized in that** it is designed as a multilayer prosthesis, preferably a two-layer prosthesis, and **in that** the phase-change material is admixed only in the layer (22) near the body.

4. Breast prosthesis (10) according to Claim 3, **characterized in that**, in the second layer near the body, the phase-change material is embedded in silicone oil, other preferably thixotropic liquids, crosslinked silicone rubber or silicone foam.

5. Breast prosthesis (10) according to Claim 1, **characterized in that** the phase-change material is designed as a panel (24) which is secured on that side of the prosthesis (10) facing towards the body.

6. Breast prosthesis (10) according to Claim 5, **characterized in that** an air cushion (26) is formed between the panel-shaped phase-change material and the prosthesis.

7. Breast prosthesis (10) according to one of the preceding claims, **characterized in that** the phase-change material is a paraffin substance.

8. Breast prosthesis (10) according to Claim 7, **characterized in that** the paraffin forming the phase-change material is used in encapsulated or stabilized form.

9. Breast prosthesis (10) according to Claim 7, **characterized in that** the paraffin used as phase-change material is used in pure form (as wax).

10. Breast prosthesis (10) according to one of Claims 5 to 9, **characterized in that** the panel-shaped material consists of a textile with integrated phase-change material.

11. Breast prosthesis (10) according to one of Claims 5 to 9, **characterized in that** the panel (24) is formed by a pouch filled with phase-change material.

## Revendications

1. Prothèse mammaire (10) constituée essentiellement de corps (12) soudés dans des feuilles de matière synthétique, reproduisant la forme du sein, en une masse de caoutchouc silicone à deux composants réticulant lors de l'addition, **caractérisée en ce qu'**elle est constituée au moins sur son côté orienté vers le corps, au moins partiellement en un matériau PCM qui présente une température de changement de phase au voisinage de la température du corps.

2. Prothèse mammaire (10) selon la revendication 1, **caractérisée en ce qu'**elle est constituée d'une couche (20), et **en ce que** le matériau PCM est ajouté à et mélangé avec la masse de caoutchouc silicone.

3. Prothèse mammaire (10) selon la revendication 1, **caractérisée en ce qu'**elle est réalisée comme prothèse multi-couches, de préférence comme prothèse à deux couches, et **en ce que** le matériau PCM est ajouté à et mélangé seulement avec la couche (22) proche du corps.

4. Prothèse mammaire (10) selon la revendication 3, **caractérisée en ce que**, dans la deuxième couche proche du corps, le matériau PCM est noyé dans de l'huile de silicone, dans d'autres liquides de préférence thixotropes, dans du caoutchouc silicone réticulé ou dans de la mousse de silicone.

5. Prothèse mammaire (10) selon la revendication 1, **caractérisée en ce que** le matériau PCM est réalisé comme plaque (24) qui est fixée sur le côté orienté vers le corps à la prothèse (10).

6. Prothèse mammaire (10) selon la revendication 5, **caractérisée en ce qu'**un coussinet d'air (26) est formé entre le matériau PCM en forme de plaque et la prothèse.

7. Prothèse mammaire (10) selon l'une des revendications précédentes, **caractérisée en ce que** le matériau PCM est une substance de paraffine.

8. Prothèse mammaire (10) selon la revendication 7, **caractérisée en ce que** la paraffine formant le matériau PCM est utilisée sous une forme encapsulée ou stabilisée.

9. Prothèse mammaire (10) selon la revendication 7, **caractérisée en ce que** la paraffine utilisée comme matériau PCM est utilisée sous forme pure (comme cire).

10. Prothèse mammaire (10) selon l'une des revendications 5 à 9, **caractérisée en ce que** le matériau en forme de plaque est réalisé en un textile dans lequel est intégré le matériau PCM.

11. Prothèse mammaire (10) selon l'une des revendications 5 à 9, **caractérisée en ce que** la plaque (24) est formée par un sachet rempli de matériau PCM.
